# FASCICULE DE BREVET EUROPEEN

(11) **EP 0 837 833 B1**
(45) Date de publication et mention de la délivrance du brevet: **25.08.1999**
(21) Numéro de dépôt: 96924013.4
(22) Date de dépôt: 24.06.1996
(51) Int. Cl.: C01F 17/00, C09D 5/08, A61K 7/48, A61K 7/42, B01J 23/10, C10M 103/06, C04B 35/50

(54) **DISPERSION COLLOIDALE ET COMPOSITION REDISPERSIBLE SOUS FORME D'UNE DISPERSION COLLOIDALE A BASE D'OXYDE DE CERIUM**
KOLLOIDALE DISPERSION UND REDISPERGIERBARE ZUSAMMENSTELLUNG EINER DISPERSION AUF CERIUMOXIDBASIS
COLLOIDALE DISPERSION AND REDISPERSIBLE COMPOSITION IN THE FORM OF A CERIUM OXIDE BASED COLLOIDAL DISPERSION

(30) Priorité: 30.06.1995 FR 9507895
(43) Date de publication de la demande: 29.04.1998
(73) Titulaire: RHODIA CHIMIE, 92408 Courbevoie Cédex (FR)
(72) Inventeur: NABAVI, Minou, F-75014 Paris (FR); CABANE, Bernard, F-75005 Paris (FR)
(74) Mandataire: Dubruc, Philippe
(86) Numéro de dépôt international: FR9600983
(87) Numéro de publication internationale: WO9702212

(56) Documents cités:
- EP-A- 0 097 563
- EP-A- 0 300 852
- EP-A- 0 304 369
- EP-A- 0 308 311
- EP-A- 0 379 814
- EP-A- 0 604 919
- FR-A- 2 416 867

## Description

La présente invention concerne une dispersion colloïdale et une composition redispersible sous forme d'une dispersion colloïdale à base d'oxyde de cérium.

Les dispersions de composés de cérium sont utilisées dans de nombreuses applications. On peut citer notamment l'utilisation en catalyse hétérogène et, en particulier, en catalyse du traitement des gaz d'échappements des moteurs à combustion interne (catalyse pour post combustion automobile). Ces dispersions peuvent aussi être utilisées comme agent de revêtement anticorrosion ou en cosmétique.

Cependant, l'utilisation de ces dispersions est limitée par une certaine instabilité dans le temps conduisant à une sédimentation. De plus, le coût du transport de ces dispersions est toujours augmenté par le fait qu'il faut transporter, outre l'élément actif, la phase liquide dans laquelle celui-ci est en suspension.

Il est, de ce fait, particulièrement intéressant de pouvoir disposer soit de dispersions stables soit de produits solides susceptibles de se redisperser pour donner une suspension colloïdale.

En outre, les procédés connus de préparation de ces dispersions ne permettent d'obtenir que des suspensions présentant un pH très acide, c'est à dire généralement inférieur à 5.

Or, dans les applications qui ont été mentionnées ci-dessus et notamment dans le cas de la cosmétique, il est important de pouvoir disposer de dispersions colloïdales ayant des pH moins acides.

Un premier objet de l'invention est donc la préparation d'une dispersion colloïdale stable d'oxyde de cérium et présentant notamment un pH élevé.

Un second objet de l'invention est l'obtention d'une composition solide à base d'oxyde de cérium qui soit redispersible sous forme d'une dispersion colloïdale.

Dans ce but, la dispersion colloïdale d'oxyde de cérium dans une phase liquide selon l'invention est caractérisée en ce qu'elle comprend en outre au moins un additif choisi parmi :
- les β dicétones;
- les monoacides carboxyliques comportant une fonction hydroxy en α ou β;
- les diols.

L'invention concerne aussi une composition redispersible sous forme d'une dispersion colloïdale, caractérisée en ce qu'elle comprend un oxyde de cérium et au moins un additif choisi parmi :
- les β dicétones;
- les monoacides carboxyliques comportant une fonction hydroxy en α ou β;
- les diols.

L'invention concerne par ailleurs un procédé de préparation d'une dispersion colloïdale d'oxyde de cérium, caractérisé en ce qu'on ajoute à une dispersion colloïdale d'oxyde de cérium de départ au moins un additif choisi parmi :
- les dicétones;
- les monoacides carboxyliques comportant une fonction hydroxy en α ou β;
- les diols.

Les dispersions selon l'invention présentent notamment l'avantage d'être stables, au moins plusieurs mois, jusqu'à des pH de l'ordre de 7,5.

D'autres caractéristiques, détails et avantages de l'invention apparaîtront encore plus complètement à la lecture de la description qui va suivre, ainsi que des divers exemples concrets mais non limitatifs destinés à l'illustrer.

Pour la suite de la description, l'expression dispersion colloïdale d'un composé de cérium désigne tout système constitué de fines particules solides de dimensions colloïdales à base d'oxyde et/ou d'oxyde hydraté (hydroxyde) de cérium en suspension dans une phase liquide, lesdites espèces pouvant en outre, éventuellement, contenir des quantités résiduelles d'ions liés ou adsorbés tels que par exemple des nitrates, des acétates, des citrates ou des ammoniums. On notera que dans de telles dispersions, le cérium peut se trouver soit totalement sous la forme de colloides, soit simultanément sous la forme d'ions et sous la forme de colloïdes.

En outre, on indique ici que le diamètre moyen des colloides doit être entendu comme désignant le diamètre hydrodynamique moyen de ces derniers, et tel que déterminé par diffusion quasi-élastique de la lumière selon la méthode décrite par Michael L. Mc CONNELL dans la revue Analytical Chemistry 53, n° 8, 1007 A, (1981).

Comme indiqué plus haut, la dispersion colloïdale de l'invention est caractérisée par la présence d'un additif qui peut tout d'abord être choisi parmi les β dicétones. Il s'agit notamment des composés de formule (1)

R₁-CO-CH2-CO-R₂ (1)

dans laquelle R₁ et R₂ peuvent être identiques ou différents et représenter un groupe alkyle, alcoxy, aryle, aryloxy, aralkyle, arylalkyloxy substitué ou non. Au moins un de R₁ et R₂ peut représenter aussi un reste de polyalkylène-éther-alcool.

On peut citer tout particulièrement les composés de formule (1) dans laquelle R₁ est un groupe alkyle, de préférence le méthyle et R₂ représente un groupe alkyle, plus particulièrement identique à R₁ ou un groupe aryle, notamment un groupe phényle.

On peut ainsi citer à titre d'exemple les composés de formule CH₃-CO-CH₂₋CO-CH₃ (acétylacétone), CH₃-CO-CH₂-CO-C₆H₆ (benzoyleacétone) ou C₆H₆-CO-CH₂-CO-C₆H₆. A titre d'exemple de reste de polyalkylène-éther-alcool, R₁ ou R₂ peut être un groupe de formule BuO(CH₂-CH₂O)ₙ.

Selon un autre mode de réalisation de l'invention, on peut choisir l'additif précité parmi les monoacides carboxyliques comportant une fonction hydroxy en α ou β.

On peut utiliser plus particulièrement les acides-phénols comme par exemple l'acide salicylique.

Selon un troisième mode de réalisation de l'invention, on peut choisir l'additif précité parmi les diols.

On choisira l'additif précité en fonction de sa nature hydrophobe ou hydrophile suivant le type de dispersion que l'on cherche à obtenir, en milieu aqueux ou en milieu solvant.

L'additif précité est habituellement présent dans une quantité telle que le rapport molaire additif/oxyde de cérium varie entre 0 (cette valeur étant exclue) et 5 et de préférence entre 0 et 1.

Le diamètre moyen des colloïdes de la dispersion est généralement d'au plus 500nm et de préférence d'au plus 10nm.

En outre, la dispersion de l'invention présente un pH compris entre 1 et 7,5, plus particulièrement entre 5 et 7,5. On entend par là que la dispersion de l'invention reste stable dans ce domaine de pH. Au delà, il peut y avoir déstabilisation de celle-ci. Toutefois, il faut noter que cette déstabilisation éventuelle n'est pas irréversible. En effet, en se ramenant à un pH dans la gamme donnée plus haut, on obtient de nouveau une dispersion stable.

La dispersion colloïdale de l'invention peut être une dispersion soit en phase aqueuse soit en phase organique. Dans ce dernier cas, la phase organique est choisie parmi celle solubilisant l'additif précité.

Dans le cas d'une dispersion en phase organique, le solvant organique utilisé peut être un hydrocarbure aliphatique ou cycloaliphatique inerte ou un mélange de ceux-ci tel que par exemple des essences minérales ou de pétrole, éthers minéraux ou de pétrole pouvant contenir également des composants aromatiques. On peut citer par exemple l'hexane, l'heptane, l'octane, le nonane, le décane, le cyclohexane, le cyclopentane, le cycloheptane et les naphtènes liquides. Les solvants aromatiques tels que le benzène, le toluène, l'éthylbenzène et les xylènes conviennent également ainsi que les coupes pétrolières du type Solvesso (marque déposée par la Société EXXON) notamment le Solvesso 100 qui contient essentiellement un mélange de méthyléthyl et triméthylbenzène et le Solvesso 150 qui renferme un mélange d'alkylbenzènes en particulier de diméthyléthylbenzène et de tétraméthylbenzène.

On peut mettre en oeuvre également des hydrocarbures chlorés tels que le chloro- ou dichlorobenzène et le chlorotoluène aussi bien que des éthers aliphatiques et cycloaliphatiques tels que l'éther de diisopropyle, l'éther de dibutyle, les cétones aliphatiques et cycloaliphatiques telles que la méthylisobutylcétone, la diisobutylcétone, l'oxyde de mésityle et les cétones phénols telle que l'acétophénone.

Les esters peuvent être envisagés. On peut citer comme esters susceptibles d'être utilisés ceux issu des alcools en C1 à C8 et notamment les palmitates d'alcool secondaire tel l'isopropanol.

Dans le cas de dispersions en phase organique, la phase liquide peut comprendre en outre un acide ou un mélange d'acides, le ou les acides pouvant être choisis parmi ceux comprenant de 11 à 50 atomes de carbone et de préférence de 15 à 25 atomes de carbone et présentant au moins une ramification en alpha, bêta, gamma ou delta de l'atome porteur de l'hydrogène acide.

Il est avantageux que le pKa d'au moins un des acides soit au plus égal à 5, de préférence à 4,5.

Il est également avantageux que la ou les chaîne(s) latérale(s) des acides ramifiés comporte au moins deux atomes, de préférence trois atomes de carbone.

A titre d'exemples, on peut citer les acides contenant du phosphore, comme les acides phosphoriques, notamment les diesters de l'acide phosphorique, les acides phosphoniques, et leurs mono esters, et les acides phosphiniques.

On peut aussi citer les acides constitutifs du mélange d'acides connu sous le nom d'acide isostéarique.

Il peut être avantageux d'ajouter dans la phase organique un agent promoteur dont la fonction est, lors de la préparation de la dispersion en phase organique, d'accélérer le transfert des colloides de la phase aqueuse à la phase organique et d'améliorer la stabilité des dispersions organiques obtenues. A titre d'agents promoteurs, on peut utiliser les composés à fonction alcool et tout particulièrement des alcools aliphatiques linéaires ou ramifiés ayant de 6 à 12 atomes de carbone.

Comme exemples spécifiques, on peut citer l'éthyl-2 hexanol, le décanol, le dodécanol ou un mélange d'entre eux.

La proportion dudit agent dans la phase organique n'est pas critique et peut varier dans de larges limites.

Toutefois une proportion comprise entre 2 et 15 % en poids convient généralement bien.

Le procédé de préparation de la dispersion de l'invention va maintenant être décrit.

On part tout d'abord d'une dispersion colloïdale qui pourra avoir été obtenue par tout moyen connu. On pourra se référer notamment aux procédés décrits dans les demandes de brevet européens EP 206906, EP 208581, EP 316205. On peut utiliser tout particulièrement les dispersions colloïdales obtenues par thermohydrolyse d'une solution aqueuse d'un sel de cérium IV, comme un nitrate, en milieu acide notamment. Un tel procédé est décrit dans la demande de brevet européen EP 239477 ou EP 208580.

On ajoute alors à cette dispersion de départ au moins un additif du type décrit plus haut et ceci dans les quantités décrites précédemment. Après addition de l'additif, il est possible de chauffer la dispersion pour accélérer la réaction.

On peut, si nécessaire, augmenter le pH de la dispersion ainsi obtenue selon différentes méthodes.

Une première méthode consiste à ajouter à la dispersion une base, comme par exemple de la soude.

Une autre méthode consiste à faire subir un traitement de dialyse à la dispersion. Dans ce cas, on effectue la dialyse contre une solution au pH désiré en utilisant une membrane de dialyse résistante au pH de la dispersion et présentant un diamètre de coupure qui soit imperméable aux colloides. Ce peut être par exemple une membrane cellulosique à paroi mince. Un avantage de cette méthode est de permettre l'obtention de dispersions à teneur faible en anion et plus particulièrement en nitrate. Ainsi, à titre d'exemple, pour les nitrates, le rapport molaire NO₃/Ce dans la dispersion est inférieur à 0,7 et peut être plus particulièrement compris entre 0,01 et 0,7 et encore plus particulièrement entre 0,15 et 0,3.

La description du procédé qui a été donnée ici s'applique à la préparation de dispersion en phase aqueuse. Pour la préparation d'une dispersion en phase organique, on prépare tout d'abord une dispersion en phase aqueuse de la manière indiquée puis on met en contact cette dispersion aqueuse avec une phase organique qui présente la composition qui a été donnée plus haut.

On peut également éliminer l'eau en séchant la dispersion aqueuse puis remettre le produit obtenu dans la phase organique.

La dispersion de l'invention permet aussi d'accéder à une composition redispersible sous forme d'une dispersion colloïdale.

Pour obtenir une telle composition on fait subir à ladite dispersion une évaporation, une centrifugation, une ultrafiltration ou une compression osmotique.

L'évaporation, la centrifugation et l'ultrafiltration peuvent se faire en utilisant tout dispositif approprié.

La compression osmotique est une méthode connu dont le principe consiste à équilibrer le potentiel chimique de l'eau à travers une membrane.

On procède en disposant la dispersion colloïdale dans un sac à dialyse par exemple en matière cellulosique, ce sac étant placé dans une solution aqueuse dont le potentiel chimique de l'eau est différent de celui de la phase aqueuse de la dispersion. Ceci peut se faire par exemple en utilisant une solution aqueuse de polyéthylène glycol (PEG) ou bien de dextran. La concentration en PEG ou en dextran fixe la pression osmotique et donc la concentration finale de la dispersion colloïdale de composé de cérium.

Les traitements qui viennent d'être mentionnés sont conduits seuls ou en combinaison et permettent de passer d'une façon continue d'une dispersion colloïdale à un gel ou pâte puis à une poudre. Cette pâte ou cette poudre peut éventuellement être séchée à une température telle qu'il n'y ait pas évaporation de l'additif décrit plus haut. La concentration en cérium peut évoluer d'une valeur de 2 à 200g/l dans la dispersion colloïdale jusqu'à une valeur de 700g/Kg, soit 1750g/l, dans la poudre ou le gel.

On obtient ainsi un composition redispersible sous forme d'une dispersion colloïdale, caractérisée en ce qu'elle comprend un oxyde de cérium et au moins un additif choisi parmi les β dicétones, les monoacides carboxyliques comportant une fonction hydroxy en α ou β et les diols.

Cette composition peut être remise en dispersion dans un milieu liquide et on obtient ainsi une dispersion colloïdale identique à la dispersion colloïdale de l'invention décrite plus haut.

Les dispersions colloïdales de l'invention telles que décrites précédemment ou celles du type obtenu par remise en dispersion d'une composition redispersible peuvent être utilisées dans de nombreuses applications. On peut citer la catalyse notamment pour post combustion automobile, la lubrification, les céramiques, la cosmétique et dans ce cas elles peuvent rentrer dans la préparation de compositions cosmétiques notamment dans la préparation de crèmes anti-UV. Elles peuvent être utilisées sur un substrat en tant qu'agent anticorrosion.

Des exemples vont maintenant être donnés.

### EXEMPLE 1

On part d'une dispersion colloïdale de CeO₂ à pH de 2. La taille des colloides est de 5nm. La concentration est de 200g/l.

On ajoute ensuite de l'acétylacétone à la dispersion dans une quantité telle que le rapport molaire acétylacétone/cérium est de 1. On obtient immédiatement une dispersion selon l'invention. On ajoute ensuite une solution de NaOH à 10% de façon à obtenir un pH de 6,5. Cette dispersion reste stable et la taille des particules définie par la méthode précitée reste identique à celle du sol initial.

Cette dispersion est concentrée par compression osmotique. La solution utilisée pour concentrer la dispersion est constituée d'une solution de dextran à 45% dans l'eau contenant une concentration d'acétylacétone de l'ordre de 10 g/l.

Lorsque l'équilibre osmotique est atteint, le produit se présente sous forme d'une pâte de concentration de 300 g/Kg, soit 400g/l.

On prend une partie de cette pâte, on l'ajoute à de l'eau désionisée. Elle se disperse spontanément pour donner une dispersion identique à la dispersion initiale.

L'autre partie de cette pâte est séchée plus complètement à l'air en présence de silicagel à une concentration de 1400 g/l, soit 650g/kg.

Elle est ensuite redispersée dans l'eau et elle donne la dispersion initiale.

### EXEMPLE 2

On ajoute à 100 ml d'une dispersion de CeO₂ de concentration en oxyde de 20 g/l et de taille de colloides de 5 nm, 0,07 g d'acide salicylique.

Cette dispersion stable est à pH 2. Une partie de cette dispersion est concentrée par compression osmotique comme précédemment. La poudre obtenue est dispersée dans une solution d'acide nitrique à pH 2.

On obtient ainsi une dispersion contenant des particules d'oxyde de cérium identiques aux particules initiales.

L'autre partie de la dispersion est dialysée contre une solution d'eau contenant 0,7 g/l d'acide salicylique dont le pH peut être fixé entre 2 et 6 à l'aide de NaOH ou bien HNO₃. On obtient ainsi une dispersion stable dont le pH est celui de la solution de dialyse.

La concentration de cette dispersion est de 20 g/l. Elle peut être augmentée par évaporation de l'eau. On prépare ainsi une poudre de concentration de 300 g/Kg, soit 400g/l, qui est partiellement redispersée dans l'eau pour obtenir une dispersion d'oxyde de cérium à pH se situant entre 2 et 6 en fonction du pH initial de la dispersion avant séchage.

### EXEMPLE 3

On utilise la dispersion d'oxyde de cérium de départ de l'exemple 2.

On en mélange 20 ml avec 0,4 g de Rhodiastab 50® (C₆H₆-CO-CH₂-CO-C₆H₆). Le mélange est mis dans un flacon fermé à l'étuve à 50°C pendant 48 h. On obtient ainsi un sol stable.

L'eau peut être éliminée par évaporation dans une étuve à 80°C. On obtient ainsi une poudre que l'on disperse dans 20 ml d'acétophénone.

On obtient ainsi une dispersion stable en milieu organique.

### EXEMPLE 4

On procède comme dans l'exemple 3 mais en utilisant 0,4 g de Rhodiastab 83® (C₆H₆-CO-CH₂-CO-CH₃).

### EXEMPLE 5

Cet exemple montre l'intérêt d'une dispersion selon l'invention dans l'application en cosmétique.

On ajoute 1g d'acétylacétone à 100ml d'une dispersion de CeO₂ à 20g/l dont la taille des colloides est de 5nm. Ensuite le pH de la dispersion est porté à 6 par dialyse contre une solution aqueuse à pH 6.

Le domaine d'absorption des rayonnements ultraviolets est mesuré à l'aide d'un spectromètre. Dans le cas de la dispersion initiale, ce domaine s'arrête à 380nm. Dans le cas de la dispersion selon l'invention, ce domaine est étendu jusqu'à 460nm.

## Revendications

1. Dispersion colloïdale d'oxyde de cérium dans une phase liquide, caractérisée en ce qu'elle comprend en outre au moins un additif choisi parmi :
- les β dicétones;
- les monoacides carboxyliques comportant une fonction hydroxy en α ou β;
- les diols.

2. Dispersion selon la revendication 1, caractérisée en ce que l'additif est choisi :
- parmi les composés de formule (1)
R₁-CO-CH2-CO-R₂ (1)
dans laquelle R₁ et R₂ peuvent être identiques ou différents et représenter un groupe alkyle, alcoxy, aryle, aryloxy, aralkyle, arylalkyloxy substitué ou non, ou encore un reste de polyalkylène-éther-alcool;
- parmi les acides-phénols.

3. Dispersion selon la revendication 1 ou 2, caractérisée en ce qu'elle présente un pH compris entre 1 et 7,5, plus particulièrement entre 5 et 7,5.

4. Dispersion selon l'une des revendications précédentes, caractérisée en ce que la phase liquide est l'eau.

5. Dispersion selon l'une des revendications précédentes, caractérisée en ce que la phase liquide est un solvant organique.

6. Dispersion selon l'une des revendications précédentes, caractérisée en ce qu'elle comprend l'additif précité dans une quantité telle que le rapport molaire additif /oxyde de cérium varie entre 0 (cette valeur étant exclue) et 5 et de préférence entre 0 et 1.

7. Procédé de préparation d'une dispersion colloidale d'oxyde de cérium, caractérisé en ce qu'on ajoute à une dispersion colloïdale d'oxyde de cérium de départ au moins un additif choisi parmi :
- les β dicétones;
- les monoacides carboxyliques comportant une fonction hydroxy en α ou β;
- les diols.

8. Procédé selon la revendication 7, caractérisé en ce qu'après addition d'au moins un additif précité, on ajoute une base à la dispersion.

9. Procédé selon la revendication 7 ou 8, caractérisé en ce qu'après addition d'au moins un additif précité, on fait subir un traitement de dialyse à la dispersion.

10. Composition redispersible sous forme d'une dispersion colloïdale, caractérisée en ce qu'elle comprend un oxyde de cérium et au moins un additif choisi parmi :
- les β dicétones;
- les monoacides carboxyliques comportant une fonction hydroxy en α ou β;
- les diols.

11. Procédé de préparation d'une composition redispersible selon la revendication précédente, caractérisé en ce qu'on part d'une dispersion du type selon l'une des revendications 1 à 6, ou du type obtenu par le procédé selon l'une des revendications 7 à 9, et on fait subir à la dite dispersion une évaporation, une centrifugation, une ultrafiltration ou une compression osmotique.

12. Utilisation d'une dispersion du type selon l'une des revendications 1 à 6 ou du type obtenu par le procédé selon l'une des revendications 7 à 9 ou du type obtenu par remise en dispersion d'une composition redispersible selon la revendication 10, sur un substrat comme agent anticorrosion, dans une composition cosmétique, en catalyse notamment pour post combustion automobile, en lubrification ou dans les céramiques.

## Patentansprüche

1. Kolloidale Dispersion von Ceriumoxid in einer flüssigen Phase, dadurch gekennzeichnet, daß sie des weiteren wenigstens ein Additiv umfaßt, das aus den
- den β-Diketonen,
- den Monocarbonsäuren, welche eine Hydroxidfunktion in α- oder β-Position tragen,
- den Diolen,
ausgewählt ist.

2. Dispersion nach Anspruch 1, dadurch gekennzeichnet, daß das Additiv aus den Verbindungen der Formel (1)
R₁-CO-CH₂-CO-R₂ (1)
worin R₁ und R₂ gleich oder verschieden voneinander sein und eine Alkyl-, Alkoxy-, Aryl-, Aryloxi-, Aralkyl-, Arylalkyloxigruppe, welche substituiert oder nicht-substituiert ist, tragen können oder auch einen Polyalkylenetheralkohol-Rest darstellen können,
- unter den Phenol säuren,
ausgewählt ist.

3. Dispersion gemäß Anspruch 1 oder 2, dadurch gekennzeichnet, daß sie einen pH-Wert von zwischen 1 und 7,5, insbesondere zwischen 5 und 7,5, zeigt.

4. Dispersion nach einem der vorhergehenden Ansprüche, dadurch gekennzeichnet, daß die wäßrige Phase Wasser ist.

5. Dispersion nach einem der vorhergehenden Ansprüche, dadurch gekennzeichnet, daß die flüssige Phase ein organisches Lösungsmittel ist.

6. Dispersion nach einem der vorhergehenden Ansprüche, dadurch gekennzeichnet, daß sie das vorgenannte Additiv in einer solchen Menge umfaßt, daß das Molverhältnis Additiv/Ceriumoxid zwischen 0 (wobei dieser Wert ausgeschlossen ist) und 5, und bevorzugt zwischen 0 und 1, variiert.

7. Verfahren zur Herstellung einer kolloidalen Dispersion von Ceriumoxid, dadurch gekennzeichnet, daß man einer anfänglichen kolloidalen Dispersion von Ceriumoxid wenigstens ein Additiv zusetzt, daß aus
- den β-Diketonen,
- den Monocarbonsäuren, welche eine Hydroxidfunktion in *α-* oder β-Position umfassen,
- den Diolen,
ausgewählt ist.

8. Verfahren nach Anspruch 7, dadurch gekennzeichnet, daß man nach der Zugabe von wenigstens einem vorgenannten Additiv der Dispersion eine Base zufügt.

9. Verfahren nach Anspruch 7 oder 8, dadurch gekennzeichnet, daß man nach der Zugabe von wenigstens einem vorgenannten Additiv die Dispersion einer Dialysebehandlung unterwirft.

10. Redispergierbare Zusammensetzung in Form einer kolloidalen Dispersion, dadurch gekennzeichnet, daß sie ein Ceriumoxid und wenigstens ein Additiv, ausgewählt aus
- den β-Diketonen,
- den Monocarbonsäuren, welche eine Hydroxidfunktion in *α-* oder β-Position umfassen,
- den Diolen,
umfaßt.

11. Verfahren zur Herstellung einer redispergierbaren Zusammensetzung nach dem vorhergehenden Anspruch, dadurch gekennzeichnet, daß man von einer Dispersion des Typs gemäß einem der Ansprüche 1 bis 6 oder des Typs, welcher durch das Verfahren nach einem der Ansprüche 7 oder 9 erhalten wird, ausgeht und man diese Dispersion einem Verdampfen, einem Zentrifugieren, einer Ultrafiltration oder einem osmotischen Druck unterwirft.

12. Verwendung einer Dispersion des Typs gemäß einem der Ansprüche 1 bis 6 oder des Typs, welcher durch das Verfahren gemäß einem der Ansprüche 7 bis 9 erhalten wird oder des Typs, welcher durch das Indispersionbringen einer redispergierbaren Zusammensetzung gemäß Anspruch 10 auf einem Substrat als Korrosionsschutzmittel, in einer kosmetischen Zusammensetzung, bei der Katalyse, insbesondere zur Nachverbrennung bei Kraftfahrzeugen, beim Schmieren oder in Keramiken.

## Claims

1. Colloidal dispersion of cerium oxide in a liquid phase, characterized in that it additionally comprises at least one additive chosen from:
- β-diketones;
- monocarboxylic acids comprising an α- or β-hydroyy functional group;
- diols.

2. Dispersion according to claim 1, characterized in that the additive is chosen;
- from the compounds of formula (1)
R₁-CO-CH₂-CO-R₂ (1)
in which R₁ and R₂ can be identical or different and can represent a substituted or unsubstituted alkyl, alkoxy, aryl, aryloxy, aralkyl or arylalkyloxy group or alternatively an alkoxypoly(alkylene oxide) residue:
- from phenol acids.

3. Dispersion according to claim 1 or 2, characterized in that it exhibits a pH of between 1 and 7.5, more particularly between 5 and 7.5.

4. Dispersion according to one of the preceding claims, characterized in that the liquid phase is water.

5. Dispersion according to one of the preceding claims, characterized in that the liquid phase is an organic solvent.

6. Dispersion according to one of the preceding claims, characterized in that it comprises the abovementioned additive in an amount such that the additive/cerium oxide molar ratio varies between 0 (this value being excluded) and 5 and preferably between 0 and 1.

7. Process for the preparation of a colloidal dispersion of cerium oxide, characterized in that at least one additive chosen from:
- β-diketones;
- monocarboxylic acids comprising an α- or β-hydroxy functional group;
- diols,
is added to a starting colloidal dispersion of cerium oxide.

8. Process according to claim 7, characterized in that, after addition of at least one abovementioned additive, a base is added to the dispersion.

9. Process according to claim 7 or 8, characterized in that, after addition of at least one abovementioned additive, the dispersion is subjected to a dialysis treatment,

10. Composition which is redispersible in the form of a colloidal dispersion, characterized in that it comprises a cerium oxide and at least one additive chosen from:
- β-diketones;
- monocarboxylic acids comprising an α- or β-hydroxy functional group;
- diols.

11. Process for the preparation of a redispersible composition according to the preceding claim, characterized in that the starting material is a dispersion of the type according to one of claims 1 to 6 or of the type obtained by the process according to one of claims 7 to 9 and the said dispersion is subjected to evaporation, centrifuging, ultrafiltration or osmotic compression.

12. Use of a dispersion of the type according to one of claims 1 to 6 or of the type obtained by the process according to one of claims 7 to 9 or of the type obtained by redispersing a redispersible composition according to claim 10 on a substrate as corrosion inhibitory, in a cosmetic composition, in catalysis, in particular for automobile afterburning, in lubrication or in ceramics.
